# EUROPEAN PATENT APPLICATION

(11) **EP 4 843 561 A1**
(43) Date of publication of application: **25.11.2026**
(21) Application number: 24918985.3
(22) Date of filing: 16.12.2024
(51) Int. Cl.: A61K 8/98, A61Q 19/08, A61Q 19/00, A61Q 7/00, A23L 33/10

(54) **COSMETIC COMPOSITION COMPRISING ORGANOID CULTURE MEDIUM**

(30) Priority: 15.01.2024 KR 20240006282
(71) Applicant: Cosmax, Inc., Hwaseong-si, Gyeonggi-do 18622 (KR); Organoidsciences Ltd., Seongnam-si, Gyeonggi-do 13493 (KR)
(72) Inventor: KYUNG, Seo Yeon, Seoul 07376 (KR); NHO, Youn Hwa, Seongnam-si, Gyeonggi-do 13486 (KR); LEE, Kyung Eun, Seongnam-si, Gyeonggi-do 13536 (KR); YUN, Seok Kyun, Seongnam-si, Gyeonggi-do 13600 (KR); KANG, Seung Hyun, Seoul 06285 (KR); NOVIANA, Wulansari, Uiwang-si, Gyeonggi-do 16016 (KR); LIM, Su Jin, Incheon 21305 (KR); YOO, Jong Man, Seongnam-si, Gyeonggi-do 13540 (KR)
(74) Representative: Rimini, Rebecca
(86) International application number: PCT/KR2024/020422
(87) International publication number: WO 2025/154961

(57) **Abstract**

The present disclosure relates to a cosmetic composition including an organoid culture medium, and since the cosmetic composition according to an aspect includes an organoid culture medium, it exhibits excellent effects such as anti-aging, moisturizing activity, promotion of hair growth, and strengthening of hair roots, and thus the cosmetic composition according to an aspect may be usefully employed for improving skin condition or improving scalp condition.

## Description

### Technical Field

The present disclosure relates to a cosmetic composition.

### Background Art

An organoid refers to an organ-specific cell aggregate produced by re-aggregating or recombining cells isolated from stem cells or organ-derived cells through a three-dimensional culture method. Organoids may be utilized in two aspects, disease models utilizing similarity with actual organs and regenerative therapeutics utilizing tissue regeneration capability.

However, an organoid culture medium is a general culture medium for differentiating organoids, and thus the applicability of the medium is limited. In addition, no study has been conducted on whether an organoid culture medium has efficacy in improving skin condition, strengthening hair roots, and promoting hair growth when applied to skin cells.

### Disclosure of Invention

### Technical Problem

One aspect is to provide a composition including an organoid culture medium.

Another aspect is to provide a cosmetic composition for improving skin condition, including an organoid culture medium.

Another aspect is to provide a health functional food composition for improving skin condition, including an organoid culture medium.

Another aspect is to provide a cosmetic composition for strengthening hair roots, including an organoid culture medium.

Another aspect is to provide a cosmetic composition for preventing hair loss or promoting hair growth, including an organoid culture medium.

Another aspect is to provide a use of an organoid culture medium for the manufacture of a cosmetic composition for improving skin condition.

Another aspect is to provide a use of an organoid culture medium for the manufacture of a health functional food composition for improving skin condition.

Another aspect is to provide a use of an organoid culture medium for the manufacture of a cosmetic composition for strengthening hair roots.

Another aspect is to provide a use of an organoid culture medium for the manufacture of a cosmetic composition for preventing hair loss or promoting hair growth.

Another aspect is to provide a method of improving skin condition, including administering an effective amount of an organoid culture medium to a subject in need thereof.

Another aspect is to provide a method of strengthening hair roots, including administering an effective amount of an organoid culture medium to a subject in need thereof.

Another aspect is to provide a method of preventing hair loss or promoting hair growth, including administering an effective amount of an organoid culture medium to a subject in need thereof.

### Solution to Problem

One aspect provides a composition including an organoid culture medium.

As used herein, the term "organoid culture medium" may be interchangeably used with "organoid culture supernatant," "organoid conditioned medium," or "organoid culture media," and may refer to an entire medium including the organoid, metabolites thereof, excess nutrients, and the like, obtained by culturing cells isolated from stem cells or organ-derived cells for a certain period of time in a medium capable of supplying nutrients so that the cells may grow, aggregate, or recombine three-dimensionally *in vitro*.

In one embodiment, the organoid culture medium may include one or more components selected from the group consisting of GlutaMAX (GLUTAMATE 1), B-27, N2, 2-mercaptoethanol, and Normocin, in a basal medium.

The basal medium may be Advanced D-MEM/F-12, Neurobasal, or a mixture thereof.

In addition, the organoid culture medium may refer to a culture medium from which organoids have been removed from a culture medium obtained by culturing the cells.

However, the liquid from which organoids are removed from the culture medium is also referred to as a "supernatant," and may be obtained by allowing the culture medium to stand for a certain period of time and collecting only the liquid from the upper layer excluding the portion settled in the lower layer, removing organoids through filtration, or centrifuging the culture medium to remove lower precipitates and collecting only the upper liquid.

The stem cells are cells having self-renewal, differentiation, and self-organization functions, and may be, for example, adult stem cells (ASC), embryonic stem cells (ESC), or induced pluripotent stem cells (iPSC). In addition, the cells isolated from the organ-derived cells may be, for example, blood, skin tissue, adipose tissue, tonsils, and the like.

In one embodiment, the organoid culture medium may be obtained before culturing cells isolated from stem cells or organ-derived cells.

In another embodiment, the organoid culture medium may be obtained after culturing cells isolated from stem cells or organ-derived cells. The organoid culture medium obtained after culturing the cells may include metabolites of the organoid. In addition, the metabolites may exhibit a synergistic effect in improving skin condition, strengthening hair roots, preventing hair loss, or promoting hair growth.

In one Example, it was confirmed that expression of moisture-related factors (AQP3 and HAS3) increased in human epidermal cells treated with an organoid culture medium obtained before or after culturing cells isolated from stem cells or organ-derived cells, and expression of skin elasticity- and wrinkle-related factors (COL1A1 and FBN1) increased in human fibroblasts. In addition, in another Example, it was confirmed that proliferation of dermal papilla cells was induced by the organoid culture medium, and expression of hair growth-related factors (VEGF, FGF7, and HGF) increased.

Accordingly, the composition according to an aspect may have an effect of improving skin condition, for example, an effect of inhibiting skin aging, improving wrinkles, improving moisturization, strengthening the skin barrier, or improving elasticity. In addition, the composition may have an effect of strengthening hair roots, preventing hair loss, or promoting hair growth.

As used herein, the term "skin aging" collectively refers to visible and invisible changes appearing in the skin as age increases, and means, for example, a decrease in epidermal thickness, and a decrease in the number of dermal cells or blood vessels, DNA damage repair capability, cell turnover cycle, wound healing, skin barrier function, moisture retention of the epidermis, sweat secretion, sebum secretion, vitamin D production, defense against physical damage, chemical removal capability, immune response, sensory function, and body temperature regulation.

The fermentation product may be for improving skin aging induced by exogenous factors or endogenous factors. The exogenous factors refer to various external factors, for example, ultraviolet rays (light), and the endogenous factors are also referred to as chronological factors and mainly refer to factors occurring due to the passage of time. That is, the skin aging specifically includes not only premature aging symptoms induced by external stimuli caused by ultraviolet rays, pollution, cigarette smoke, chemicals, and the like, but also natural aging phenomena occurring due to decreased proliferation of skin cells as age increases, and is a concept including wrinkles, reduced elasticity, skin sagging, and dryness. In addition, wrinkles include the induction of wrinkles by stimuli caused by changes in internal or external factors changing components constituting skin tissue.

The aging may be photoaging. The term "photoaging" refers to a phenomenon induced by external environmental factors, and the most representative factor is ultraviolet rays. Ultraviolet rays cause damage to biological components such as activation of proteolytic enzymes, chain cleavage of matrix proteins, and abnormal crosslinking, and repetition of such mechanisms results in visibly apparent skin aging.

As used herein, the term "wrinkle" refers to a loosened state caused by loss of skin elasticity, and may be, for example, folding of the skin. Pigmentation refers to a state in which the amount of pigment in the body is abnormal or there is an abnormality in the location where pigment appears, and may be, for example, melasma and freckles. The "prevention or improvement of skin wrinkles" may refer to any action of preventing or improving wrinkles by suppressing expression of wrinkle-related factors or increasing the total amount of collagen.

The "strengthening of the skin barrier" may refer to any action in which the function of the skin barrier positioned at the outermost layer of the skin and preventing loss of moisture and nutrients is enhanced.

The "skin moisturization" may refer to any action of maintaining skin moisture or preventing moisture loss.

The "skin regeneration" may refer to any action of supplementing a portion of the skin when a part of the skin is lost or damaged, or promoting proliferation and migration of skin cells.

The composition may include 0.001 wt% to 80 wt%, for example, 0.01 wt% to 60 wt%, 0.01 wt% to 40 wt%, 0.01 wt% to 30 wt%, 0.01 wt% to 20 wt%, 0.01 wt% to 10 wt%, 0.01 wt% to 5 wt%, 0.05 wt% to 60 wt%, 0.05 wt% to 40 wt%, 0.05 wt% to 30 wt%, 0.05 wt% to 20 wt%, 0.05 wt% to 10 wt%, 0.05 wt% to 5 wt%, 0.1 wt% to 60 wt%, 0.1 wt% to 40 wt%, 0.1 wt% to 30 wt%, 0.1 wt% to 20 wt%, 0.1 wt% to 10 wt%, or 0.1 wt% to 5 wt% of an organoid culture medium based on the total weight of the composition. At this time, when the content of the organoid culture medium is less than the above range, there is a problem in that effects of improving skin condition such as anti-aging and moisturization or effects of strengthening hair roots are not sufficiently exhibited.

As used herein, the term "included as an active ingredient" means that the organoid culture medium of the present disclosure is added in an amount sufficient to exhibit the effects described above, and includes formulation into various forms by additionally adding various components as auxiliary ingredients for drug delivery, stabilization, and the like.

In one embodiment, the composition may be a cosmetic composition.

The cosmetic composition may have, for example, a cosmetic formulation type of skin softener, nourishing toner, massage cream, nourishing cream, essence, pack, gel, ampoule, or skin adhesive-type cosmetic formulation.

Components included in the cosmetic composition may include, as active ingredients, in addition to the above composition, components commonly used in cosmetic compositions, and may include, for example, general adjuvants and carriers such as stabilizers, solubilizers, vitamins, pigments, and fragrances.

In another embodiment, the composition may be a pharmaceutical composition.

The pharmaceutical composition may additionally include a pharmaceutically acceptable diluent or carrier. The diluent may be lactose, corn starch, soybean oil, microcrystalline cellulose, mannitol, and a lubricant may be magnesium stearate, talc, or a combination thereof. The carrier may be an excipient, disintegrant, binder, lubricant, or a combination thereof. The excipient may be microcrystalline cellulose, lactose, low-substituted hydroxycellulose, or a combination thereof. The disintegrant may be calcium carboxymethyl cellulose, sodium starch glycolate, anhydrous dibasic calcium phosphate, or a combination thereof. The binder may be polyvinylpyrrolidone, low-substituted hydroxypropyl cellulose, hydroxypropyl cellulose, or a combination thereof. The lubricant may be magnesium stearate, silicon dioxide, talc, or a combination thereof.

The pharmaceutical composition may be formulated as an oral administration formulation or a parenteral administration formulation. The oral administration formulation may be granules, powders, liquid formulations, tablets, capsules, dry syrups, or a combination thereof. The parenteral administration formulation may be an injectable formulation.

In another embodiment, the composition may be a composition for external application to skin.

The preparation for external application to skin may be cream, gel, ointment, skin emulsion, skin suspension, transdermal delivery patch, drug-containing bandage, lotion, or a combination thereof. The preparation for external application to skin may be appropriately blended, as necessary, with components commonly used in compositions for external application to skin such as cosmetics or pharmaceuticals, for example, aqueous components, oily components, powder components, alcohols, moisturizers, thickeners, ultraviolet absorbers, whitening agents, preservatives, antioxidants, surfactants, fragrances, colorants, various skin nutrients, or a combination thereof. The preparation for external application to skin may also be appropriately blended with metal sequestrants such as disodium edetate, trisodium edetate, sodium citrate, sodium polyphosphate, sodium metaphosphate, and gluconic acid; agents such as caffeine, tannin, verapamil, licorice extract, glabridin, hot water extract of Karin fruit, various herbal medicines, tocopheryl acetate, glycyrrhetinic acid, tranexamic acid and derivatives or salts thereof; and sugars such as vitamin C, magnesium ascorbyl phosphate, ascorbyl glucoside, arbutin, kojic acid, glucose, fructose, and trehalose may also be appropriately blended.

In another embodiment, the composition may be a health functional food composition.

The health functional food composition may be used alone or together with other foods or food ingredients, and may be appropriately used according to general methods. The mixing amount of the active ingredient may be appropriately determined depending on the purpose of use (prevention, health, or therapeutic treatment). In general, during manufacture of food or beverages, the composition of the present disclosure may be added in an amount of 15 parts by weight or less based on the raw material. There is no particular limitation on the type of the health functional food. Among the types of health functional food, a beverage composition may include, as additional components, various flavoring agents or natural carbohydrates, as in general beverages. The natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As sweeteners, natural sweeteners such as thaumatin and stevia extract, or synthetic sweeteners such as saccharin and aspartame may be used. The health functional food composition may further include nutrients, vitamins, electrolytes, flavoring agents, colorants, pectic acid and salts thereof, alginic acid and salts thereof, organic acids, protective colloid thickeners, pH regulators, stabilizers, preservatives, glycerin, alcohol, carbonating agents used in carbonated beverages, or a combination thereof. The health functional food composition may further include natural fruit juice, fruit juice beverage, pulp for production of vegetable beverages, or a combination thereof.

Another aspect provides a method of preventing, improving, or treating a skin condition of a subject, including treating or administering an effective amount of the composition or the organoid culture medium to a subject in need thereof. Specific details of the skin condition are as described above.

As used herein, the terms "administering," "introducing," and "transplanting" are used interchangeably and may refer to placement of the composition according to an embodiment into a subject by a method or route resulting in at least partial localization to a desired site of the composition according to an embodiment.

Administration may be carried out by methods known in the art. Administration may be directly performed to the subject by any means through routes such as, for example, intravenous, intramuscular, oral, transdermal, mucosal, intranasal, intratracheal, or subcutaneous administration. The administration may be systemic or local administration.

The subject may be a mammal, for example, a human, cow, horse, pig, dog, sheep, goat, or cat. The subject may be a subject requiring improvement of skin cosmetic condition, for example, skin moisturization, strengthening of the skin barrier, or wrinkle improvement effects.

The administration may include administering the composition according to an embodiment in an amount of 0.1 mg to 1,000 mg per subject per day, for example, 0.1 mg to 500 mg, 0.1 mg to 100 mg, 0.1 mg to 50 mg, 0.1 mg to 25 mg, 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg. However, the dosage may be variously prescribed depending on factors such as formulation method, administration method, age, body weight, sex, pathological condition, food, administration time, administration route, excretion rate, and response sensitivity of the patient, and those skilled in the art may appropriately adjust the dosage in consideration of these factors. The frequency of administration may be once per day or two or more times within a clinically acceptable range of side effects, and administration may be performed at one site or two or more sites, and may be administered daily or at intervals of 2 to 5 days, with the total administration period ranging from 1 day to 30 days for a single treatment. If necessary, the same treatment may be repeated after an appropriate period of time. For animals other than humans, administration may be performed at the same dosage as humans on a per kg basis, or alternatively, an amount converted from the above dosage based on, for example, the volume ratio (for example, an average value) of organs (heart, etc.) between the target animal and a human may be administered.

Another aspect provides a method of strengthening hair roots, including administering an effective amount of the composition or the organoid culture medium to a subject in need thereof.

Another aspect provides a method of preventing hair loss or promoting hair growth, including administering an effective amount of the composition or the organoid culture medium to a subject in need thereof.

Another aspect provides a use of an organoid culture medium for the manufacture of a cosmetic composition for improving skin condition.

Another aspect provides a use of an organoid culture medium for the manufacture of a health functional food composition for improving skin condition.

Another aspect provides a use of an organoid culture medium for the manufacture of a cosmetic composition for strengthening hair roots.

Another aspect provides a use of an organoid culture medium for the manufacture of a cosmetic composition for preventing hair loss or promoting hair growth.

Since the method or use includes or utilizes the above-described composition or organoid culture medium as it is, descriptions common therebetween are omitted in order to avoid excessive complexity of the present disclosure.

### Advantageous Effects of Invention

The organoid culture medium according to an aspect has excellent anti-aging, moisturizing activity, and hair root strengthening effects, and thus may be usefully used for improving skin condition or improving scalp condition.

### Brief Description of Drawings

FIG. 1 illustrates the effect of an organoid culture medium according to an embodiment on expression of AQP3.
FIG. 2 illustrates the effect of an organoid culture medium according to an embodiment on expression of HAS3.
FIG. 3 illustrates the effect of an organoid culture medium according to an embodiment on expression of COL1A1.
FIG. 4 illustrates the effect of an organoid culture medium according to an embodiment on expression of FBN.
FIG. 5 illustrates the effect of an organoid culture medium according to an embodiment on cell proliferation of dermal papilla cells.
FIG. 6 illustrates the effect of an organoid culture medium according to an embodiment on expression of VEGF.
FIG. 7 illustrates the effect of an organoid culture medium according to an embodiment on expression of FGF.

### Mode for the Invention

Hereinafter, preferred Examples are provided in order to facilitate understanding of the present disclosure. However, the following Examples are provided only to facilitate understanding of the present disclosure, and the scope of the present disclosure is not limited by the following Examples.

### [Example]

### Example 1. Preparation of Organoid Culture Medium (1)

An organoid culture medium according to an aspect was prepared. Specifically, an organoid culture media was prepared by mixing X100 GLUTAMATE 1, B-27 supplement, N2 supplement, 2-Mercaptoethanol, and Normocin in Advanced D-MEM/F-12 and Neurobasal media.

### Example 2. Preparation of Organoid Culture Medium (2)

An organoid culture medium according to an aspect was prepared. Specifically, hESC or iPSC cells were cultured in 500 µl of the medium of Example 1 in a 24-well plate while the existing medium was removed and replaced with fresh medium once every 3 days , thereby preparing organoids. After culturing for 100 to 200 days, organoids in which hair had formed were transferred into the medium of Example 1 and additionally cultured for 3 days. Thereafter, the organoid culture medium was collected, and centrifuged to precipitate cells and debris, and only the supernatant was obtained.

### [Experimental Example]

### Experimental Example 1. Confirmation of Skin Moisturization Improvement Effect

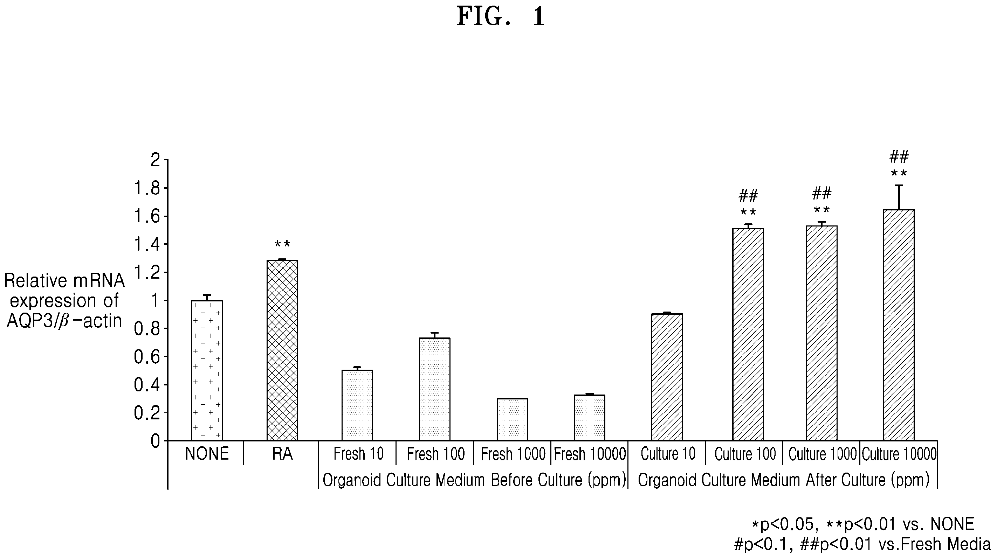

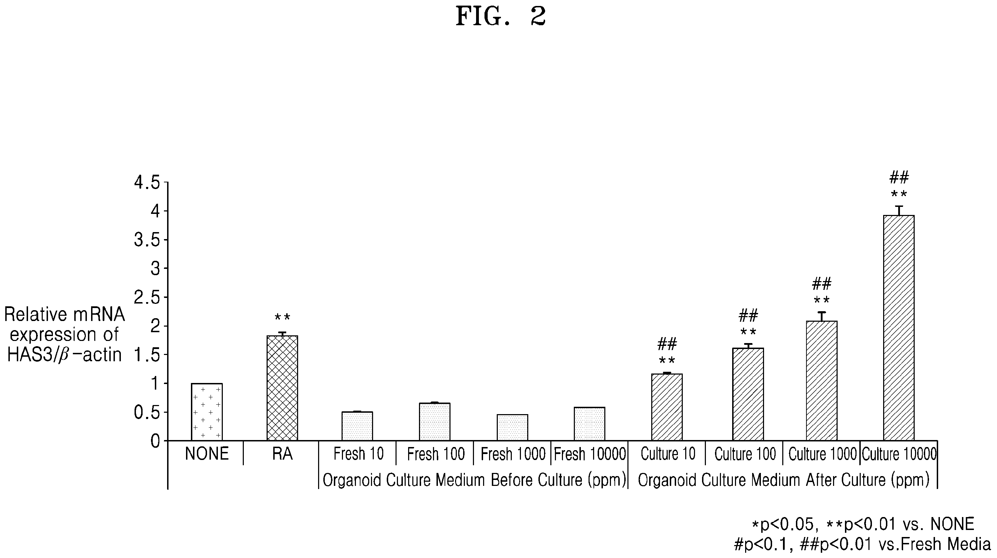

The skin moisturization improvement effect of the organoid culture medium according to an aspect was confirmed. Specifically, human epidermal cells (HaCaT) were cultured in Dulbecco's modified Eagle's medium (DMEM) including 10 % fetal bovine serum (FBS) and 1 % penicillin, and culturing was performed for 24 hours in an incubator under conditions of 37 °C, 5 % CO₂, and 95 % humidity. Thereafter, the medium was removed, DPBS was added, and washing was performed once, after which the medium was replaced with an FBS-free medium, and Example 1 and Example 2 were treated at respective concentrations (10, 100, 1000, and 10000 ppm) and additionally cultured for 24 hours. Pure distilled water was used as a negative control group, and a group treated with 1 µM retinoic acid was used as a positive control group. Thereafter, cells from each medium were collected and washed with cooled PBS, RNA was isolated using an RNAprep kit (Monarch^{®} Total RNAprep Kit, NEB, USA), RNA was quantified at 260 nm using a nanodrop, and cDNA was synthesized in a thermal cycler using 2 µg of RNA for each sample (C1000 Thermal Cycler, Bio-Rad, USA). Using the synthesized cDNA as a template, real-time polymerase chain reaction was performed for target genes AQP3 and HAS3 by adding SYBR Green Supermix (Applied Biosystems, USA) together with primers and cDNA in a real-time PCR instrument (Step One Plus, Applied Biosystems, USA). The real-time PCR reaction was performed by activating polymerase at 94 °C for 5 minutes, followed by repeating 40 cycles of reactions at 95 °C for 30 seconds, 60 °C for 30 seconds, and 72 °C for 30 seconds. Expression levels of the AQP3 and HAS3 genes were finally analyzed through normalization with respect to the β-actin gene, and the results are illustrated in FIGS. 1 and 2.

FIG. 1 illustrates the effect of an organoid culture medium according to an embodiment on expression of AQP3.

FIG. 2 illustrates the effect of an organoid culture medium according to an embodiment on expression of HAS3.

As a result, as illustrated in FIG. 1 and FIG. 2, it was confirmed that Example 2 increased expression of AQP3 and HAS3 in a concentration-dependent manner. Specifically, it was confirmed that Example 2 significantly increased expression of AQP3 at a concentration of 100 ppm or more as compared with the positive control group, and increased expression of HAS3 at a concentration of 1000 ppm as compared with the positive control group.

Accordingly, the organoid culture medium according to an embodiment is effective for skin moisturization by increasing expression of AQP3 and HAS3.

### Experimental Example 2. Confirmation of Skin Elasticity Effect

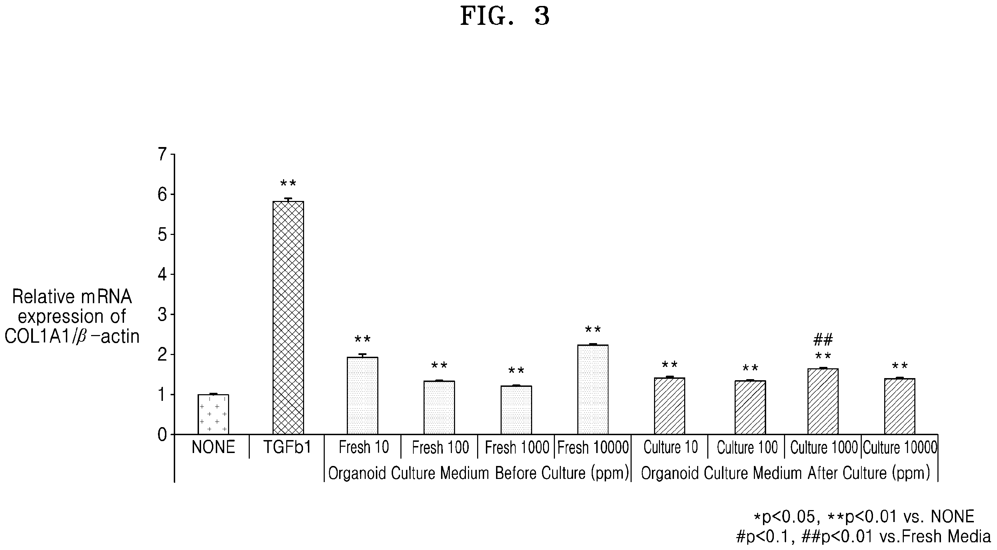

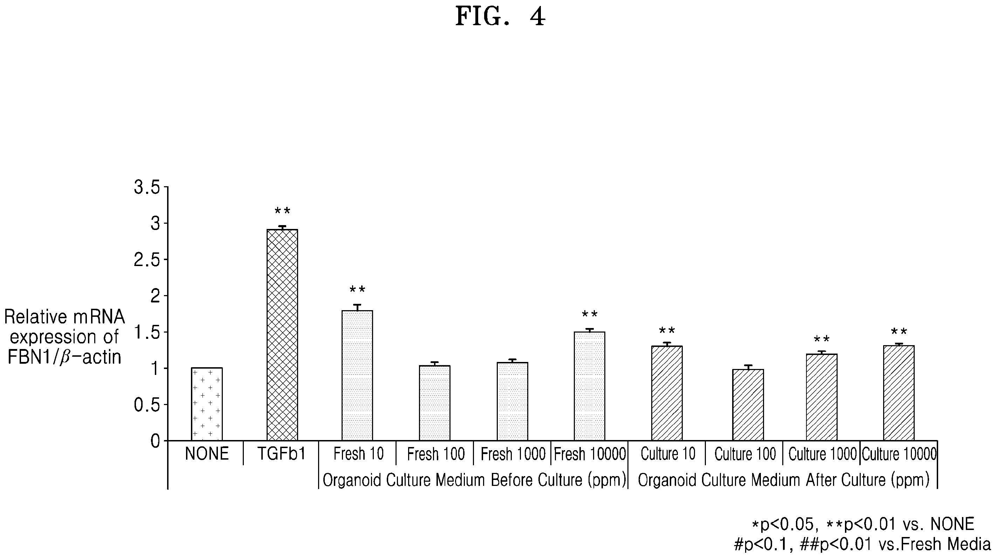

The skin elasticity and wrinkle improvement effects of the organoid culture medium according to an aspect were confirmed. Specifically, a human dermal fibroblast cell line (Hs68) was cultured in Dulbecco's modified Eagle's medium (DMEM) including 10 % fetal bovine serum (FBS) and 1 % penicillin, and culturing was performed for 24 hours in an incubator under conditions of 37 °C, 5 % CO₂, and 95 % humidity. Thereafter, the medium was removed, DPBS was added, and UVB at 20 mJ/cm² was irradiated or not irradiated. Immediately after UVB irradiation, DPBS was removed and the medium was replaced with an FBS-free medium, after which Example 1 and Example 2 were treated at respective concentrations (10, 100, 1000, and 10000 ppm) and additionally cultured for 24 hours. Pure distilled water was used as a negative control group, and a group treated with 1 nM TGF-beta1 was used as a positive control group. Thereafter, cells from each medium were collected and washed with cooled PBS, RNA was isolated using an RNAprep kit (Monarch^{®} Total RNAprep Kit, NEB, USA), RNA was quantified at 260 nm using a nanodrop, and cDNA was synthesized in a thermal cycler using 2 µg of RNA for each sample (C1000 Thermal Cycler, Bio-Rad, USA). Using the synthesized cDNA as a template, real-time polymerase chain reaction was performed for target genes COL1A1 and FBN1 by adding SYBR Green Supermix (Applied Biosystems, USA) together with primers and cDNA in a real-time PCR instrument (Step One Plus, Applied Biosystems, USA). The real-time PCR reaction was performed by activating polymerase at 94 °C for 5 minutes, followed by repeating 40 cycles of reactions at 95 °C for 30 seconds, 60 °C for 30 seconds, and 72 °C for 30 seconds. Expression levels of the COL1A1 and FBN1 genes were finally analyzed through normalization with respect to the β-actin gene, and the results are respectively illustrated in FIG. 3 and FIG. 4.

FIG. 3 illustrates the effect of an organoid culture medium according to an embodiment on expression of COL1A1.

FIG. 4 illustrates the effect of an organoid culture medium according to an embodiment on expression of FBN1.

As a result, as illustrated in FIG. 3 and FIG. 4, it was confirmed that Example 1 and Example 2 increased expression of COL1A1 and FBN1 as compared with the untreated control group.

Accordingly, the organoid culture medium according to an embodiment has an excellent effect on skin elasticity by increasing expression of COL1A1 and FBN1 regardless of whether it is obtained before culturing the cells or after culturing the cells.

### Experimental Example 3. Confirmation of Cell Proliferation

The effect of the organoid culture medium according to an aspect on cell proliferation of dermal papilla cells was confirmed. Specifically, HFDPC were seeded in a 96-well plate at a density of 2 x 10⁴ cells and then cultured for 24 hours in an incubator under conditions of 37 °C and 5 % CO₂. Thereafter, the medium was replaced with a medium containing no components, the organoid culture medium was treated, and additional culturing was performed for 24 hours. Minoxidil, which is a positive control group, was used, and after 24 hours, the medium in each well was removed, followed by one washing process using Dulbecco's phosphate buffered saline (DPBS), after which the medium was replaced with a medium including 500 µl per well of 0.5 mg/mL MTT tetrazolium (Sigma-Aldrich, St. Louis, USA), and reacted at 37 °C for 4 hours. Thereafter, absorbance was measured at 570 nm using Victor 3. HFDPC proliferation ability was measured as a percentage relative to the average absorbance value thereof by setting 0.1 µM minoxidil (MXD), which is known as a hair growth promoter, as a positive control group, and setting a case in which a medium containing no components was treated as a negative control group for cell viability.

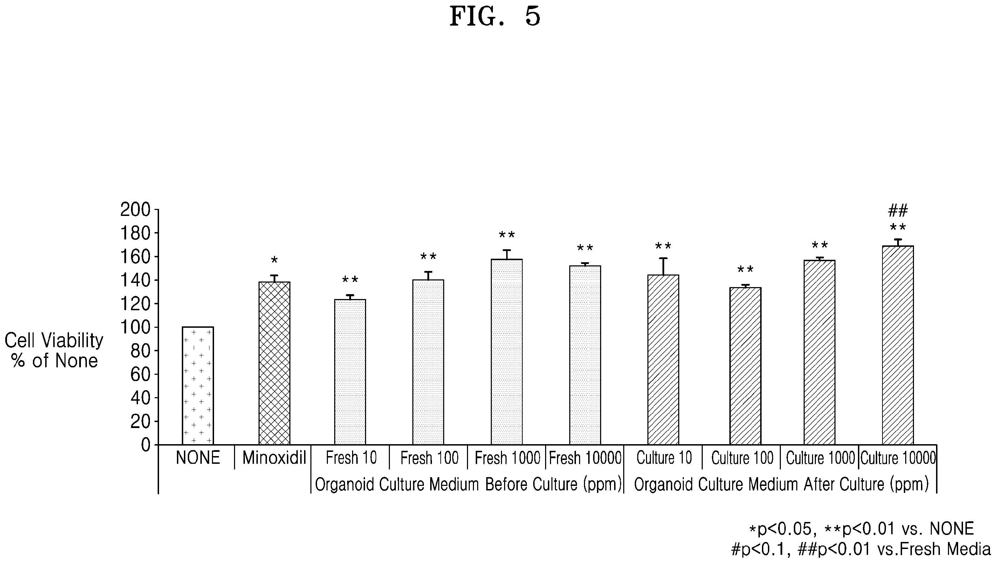

FIG. 5 illustrates the effect of an organoid culture medium according to an embodiment on cell proliferation of dermal papilla cells.

As a result, as illustrated in FIG. 5, it was confirmed that Example 1 and Example 2 promoted cell proliferation of dermal papilla cells as compared with the untreated control group. Specifically, it was confirmed that Example 1 and Example 2 had a positive effect on cell proliferation of dermal papilla cells similar to or greater than minoxidil.

Accordingly, the organoid culture medium according to an embodiment promotes cell proliferation of dermal papilla cells regardless of whether it is obtained before culturing the cells or after culturing the cells, and thus may be usefully employed for promotion or improvement of hair growth, or prevention, improvement, or treatment of hair loss.

### Experimental Example 4. Confirmation of Hair Growth Effect

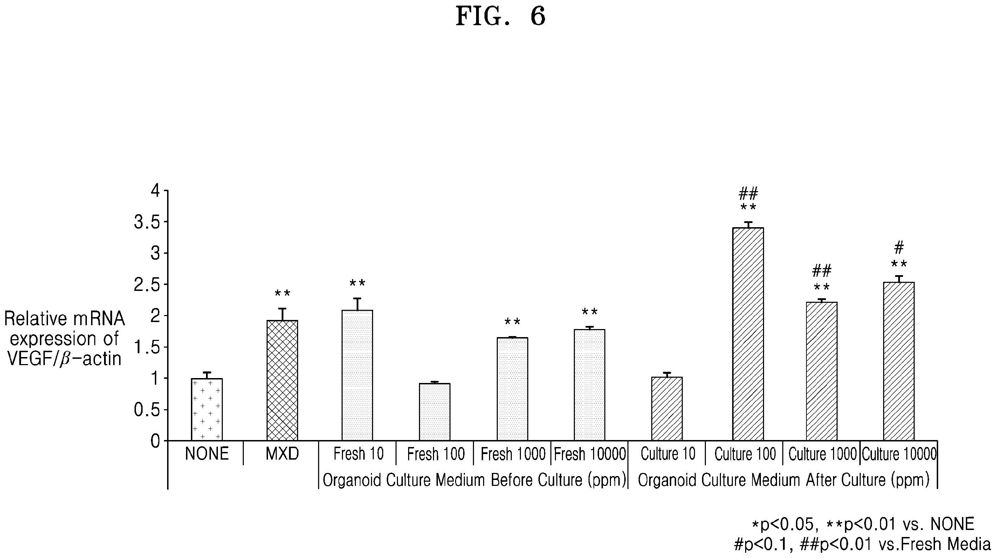

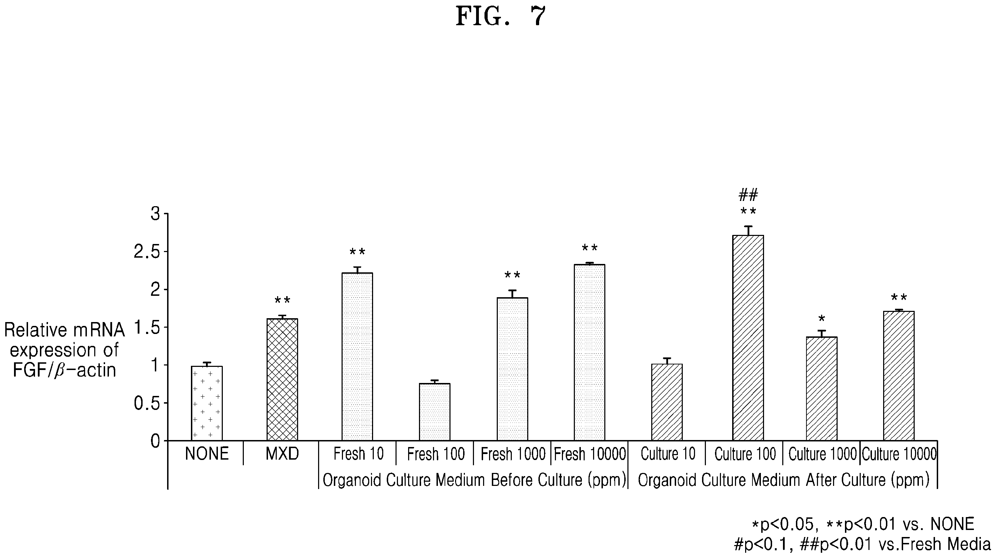

The hair growth effect of the organoid culture medium according to an aspect was confirmed. Specifically, human follicle dermal papilla cells (HFDPC) were seeded in a 6-well plate at 3 x 10⁵ cells/well and cultured for 24 hours under cell culture conditions. After 24 hours, the medium was discarded and washed with PBS, after which the cells were starved using a medium not including FBS, and on the following day, the medium was replaced with an FBS-free medium and a medium including 0.1 µM minoxidil, Example 1, or the organoid culture medium of Example 2, followed by additional culturing for 24 hours. Thereafter, RNA was isolated from the cells using an RNeasy Mini Kit (Qiagen), RNA was quantified at 260 nm using a nanodrop, and cDNA was synthesized in a thermal cycler (C1000 Thermal Cycler, Bio-Rad, USA) using 2 µg of RNA for each sample. Using a mixture obtained by adding target proteins VEGF and FGF7, primers, and the cyanine dye SYBR Green Supermix (Applied Biosystems, USA) to the synthesized cDNA, real-time polymerase chain reaction was performed in a real-time PCR instrument, thereby finally evaluating expression levels of VEGF and FGF7 genes. The real-time PCR reaction was performed by activating polymerase at 94 °C for 5 minutes, followed by repeating 40 cycles of reactions at 95 °C for 30 seconds, 60 °C for 30 seconds, and 72 °C for 30 seconds. Expression levels of the VEGF and FGF genes were finally analyzed through normalization with respect to the β-actin gene, and the results are respectively illustrated in FIG. 6 and FIG. 7.

FIG. 6 illustrates the effect of an organoid culture medium according to an embodiment on expression of VEGF.

FIG. 7 illustrates the effect of an organoid culture medium according to an embodiment on expression of FGF.

The foregoing description of the present disclosure is for illustrative purposes, and those skilled in the art will understand that various modifications into other specific forms may be readily made without changing the technical concept or essential characteristics of the present disclosure. Accordingly, it should be understood that the embodiments described above are illustrative in all respects and not restrictive.

## Claims

1. A cosmetic composition for improving skin condition, comprising an organoid culture medium.

2. The cosmetic composition of claim 1, wherein the organoid culture medium is obtained before culturing cells isolated from stem cells or organ-derived cells or after culturing the cells.

3. The cosmetic composition of claim 1, wherein the skin condition is inhibition of aging, wrinkle improvement, moisturization improvement, strengthening of the skin barrier, or elasticity improvement.

4. The cosmetic composition of claim 3, wherein the aging is aging caused by ultraviolet rays.

5. A health functional food composition for improving skin condition, comprising an organoid culture medium.

6. A cosmetic composition for strengthening hair roots, comprising an organoid culture medium.

7. A cosmetic composition for preventing hair loss or promoting hair growth, comprising an organoid culture medium.

8. Use of an organoid culture medium for the manufacture of a cosmetic composition for improving skin condition.

9. Use of an organoid culture medium for the manufacture of a health functional food composition for improving skin condition.

10. Use of an organoid culture medium for the manufacture of a cosmetic composition for strengthening hair roots.

11. Use of an organoid culture medium for the manufacture of a cosmetic composition for preventing hair loss or promoting hair growth.

12. A method of improving skin condition, comprising administering an effective amount of an organoid culture medium to a subject in need thereof.

13. A method of strengthening hair roots, comprising administering an effective amount of an organoid culture medium to a subject in need thereof.

14. A method of preventing hair loss or promoting hair growth, comprising administering an effective amount of an organoid culture medium to a subject in need thereof.
